# EUROPÄISCHE PATENTSCHRIFT

(11) **EP 2 915 502 B1**
(45) Veröffentlichungstag und Bekanntmachung des Hinweises auf die Patenterteilung: **20.11.2019**
(21) Anmeldenummer: 14157978.9
(22) Anmeldetag: 06.03.2014
(51) Int. Cl.: A61C 1/00, A61B 5/00, H04B 13/00

(54) **Medizinisches, insbesondere zahnärztliches, System**
Medical, in particular dental system
Système médical, notamment de dentisterie

(43) Veröffentlichungstag der Anmeldung: 09.09.2015
(60) Teilanmeldung: 19195832.1
(73) Patentinhaber: W & H Dentalwerk Bürmoos GmbH, 5111 Bürmoos (AT)
(72) Erfinder: SCHRÖCK, Rainer, 5111 Bürmoos (AT); TANNEBAUM, Wolfgang, 92637 Weiden (DE)

(56) Entgegenhaltungen:
- EP-A1- 1 187 375
- EP-A2- 0 843 425
- DE-A1-102012 105 365
- US-A- 3 753 434
- US-A1- 2010 249 509

## Beschreibung

Die vorliegende Erfindung bezieht sich auf ein medizinisches, insbesondere zahnärztliches, System mit zumindest einer Sendeeinheit und zumindest einer Empfangseinheit nach dem Oberbegriff des Anspruchs 1.

Derartige medizinische, insbesondere zahnärztliche, Systeme dienen bevorzugt zur Bearbeitung von menschlichen oder tierischen Gewebe. Hierzu weisen diese bevorzugt zumindest ein medizinisches, insbesondere zahnärztliches, Hand- oder Winkelstück, eine mit dem Hand- oder Winkelstück verbindbare oder verbundene Antriebs- und/ oder Steuereinheit, sowie zumindest ein für den Betrieb des Hand- oder Winkelstücks und/ oder der Antriebs und/ oder Steuereinheit ausgebildetes Zubehör auf.

Das zumindest eine Hand- oder Winkelstück ist hierbei bevorzugt zum Antreiben eines auf eine Behandlungsstelle einwirkenden Werkzeugs ausgebildet. Das Werkzeug wird bevorzugt mittels eines Elektromotors, eines Luftantriebs oder eines Piezoantriebs, welcher vorzugsweise in dem Hand- oder Winkelstück angeordnet ist oder mit diesem, insbesondere über eine Kupplungsvorrichtung, verbunden ist, in Bewegung versetzt. Des Weiteren kann das zumindest eine Hand- oder Winkelstück zum Abgeben von Strahlung, Fluiden, Energie, von abrasiven Medien und/ oder zur Bereitstellung von Messdaten ausgebildet sein.

Um das medizinische Hand- oder Winkelstück anzutreiben und/ oder anzusteuern, ist dieses bevorzugt mittels einer Versorgungsleitung mit der Antriebs- und/ oder Steuereinheit verbunden. Die Antriebs- und/ oder Steuereinheit stellt die für das medizinische Hand- oder Winkelstück und/ oder Werkzeug jeweils benötigte Arbeitsmedien, wie zum Beispiel Sprayluft und/ oder Spraywasser zur Kühlung, Antriebsfluid, insbesondere Druckluft, Strahlung oder elektrische Energie zur Versorgung von in dem Hand- oder Winkelstück angeordneten elektrischen Bauteilen, wie z.B. von einer oder mehreren Leuchtdioden zur Beleuchtung der Behandlungsstellen, Sensoren oder von elektrischen Speichern zur Speicherung von bevorzugt instrumentenbezogenen Daten, zur Verfügung.

Des Weiteren weisen die medizinischen Systeme bevorzugt zumindest ein zum Betrieb des Hand- oder Winkelstücks und/ oder ein für den Betrieb der Antriebs- und/ oder Steuereinheit ausgebildetes Zubehör auf. Beispiele hierfür sind: Instrumententräger zur Aufbewahrung und Lagerung der medizinischen Hand- oder Winkelstücke oder der Werkzeuge, Brillen, insbesondere Schutzbrillen oder Filterbrillen zur Erkennung einer Anomalie an einem Zahn, Handspiegel, Fußanlasser zur Betätigung der Antriebs- und/ oder Steuereinheit, mit der Antriebs- und oder Steuereinheit kabelgebunden oder kabellos verbundene Anzeigeelemente, oder Mittel zur Benutzererkennung, wie zum Beispiel Magnetkarten oder Chipkarten.

Zur Aufbereitung, insbesondere zur Reinigung, Desinfektion und/ oder Sterilisation, des zumindest einen medizinischen, insbesondere zahnärztlichen, Hand- oder Winkelstücks sowie auch zur Aufbereitung des Zubehörs des medizinischen Systems, stehen dem Anwender Aufbereitungsvorrichtungen zur Verfügung. Diese Sterilisatoren, Autoklaven oder Thermodesinfektoren leiten hierzu zumindest ein Betriebsmedium in eine Aufbereitungskammer, in der das zumindest eine medizinische, insbesondere zahnärztliche, Hand- oder Winkelstück oder Zubehör, vorzugsweise mittels eines Aufbereitungsträgers, aufgenommen ist, oder führen das Medium dem zumindest einen medizinischen Hand- oder Winkelstück direkt zu. Als Betriebsmedien können vorzugsweise Flüssigkeiten, wie zum Beispiel Heißwasser, oder Dämpfe, insbesondere gesättigter Wasserdampf, verwendet werden.

Im Stand der Technik ist es nun bekannt das zumindest eine Hand- oder Winkelstück und/ oder Zubehör des medizinischen Systems mit einer Codierung zu versehen, um die Arbeitsmedien der Antriebs- und/ oder Steuereinheit und/ oder die Betriebsmedien der Aufbereitungsvorrichtung dem Hand- oder Winkelstück automatisch, abhängig von dem über den Versorgungsschlauch mit der Antriebs- und/ oder Steuereinheit verbundenen oder in der Aufbereitungsvorrichtung angeordneten Hand- oder Winkelstück oder Zubehör des medizinischen Systems, zuzuführen. Hierzu enthält die Codierung insbesondere Identifizierungsdaten, wie z.B. eine Seriennummer, welche mittels einer Lesevorrichtung von der Antriebs- und/ oder Steuereinheit und/ oder von der Aufbereitungsvorrichtung auslesbar ist.

Ein derartiges medizinisches, insbesondere zahnärztliches, System ist insbesondere aus der EP 1 392 193 B1 bekannt.

Dieses bekannte medizinische System umfasst ein Versorgungsgerät zum Versorgen zumindest eines medizinischen Hand- oder Winkelstücks mit Medien, insbesondere mit Wasser, Druckluft oder Licht, wobei sich eine Versorgungsleitung von dem Versorgungsgerät erstreckt, an deren Ende ein Kupplungsteil angeordnet ist, das mit einem korrespondierenden Kupplungsteil des Hand- oder Winkelstücks kuppelbar ist. Im Inneren des Hand- oder Winkelstücks ist ein Speicherelement für Identifikationsdaten vorgesehen und für das korrespondierende Kupplungsteil der Versorgungsleitung zugänglich. Das Auslesen des Speicherelements, insbesondere die Übertragung der Daten von dem Hand- oder Winkelstück auf das Versorgungsgerät, erfolgt entweder durch eine mechanische Kontaktierung mittels elektrischer Kontakte in den beiden Kupplungsteilen oder durch eine drahtlose Kontaktierung mittels zweier Transponder.

Des Weiteren umfasst das bekannte medizinische System eine Aufbereitungsvorrichtung mit zumindest einem Kupplungsteil, welches ebenfalls mit dem korrespondierenden Kupplungsteil des Hand- oder Winkelstücks kuppelbar ist. Das Auslesen des Speicherelements in dem Hand- oder Winkelstück erfolgt auch hier durch eine mechanische Kontaktierung mittels elektrischer Kontakte oder durch eine drahtlose Kontaktierung mittels zweier Transponder, welche im Kupplungsbereich der beiden Kupplungsteile angeordnet sind.

Als nachteilig dieser Ausgestaltung des medizinischen Systems erweist sich die Übertragung der elektrischen Signale, insbesondere der Daten, zwischen dem Hand-oder Winkelstück und dem Versorgungsgerät und/ oder der Aufbereitungsvorrichtung. Eine Übertragung von Signalen und/ oder Daten ist nur in einem gekoppelten Zustand des Hand- oder Winkelstücks mit dem Versorgungsgerät und/ oder mit der Aufbereitungsvorrichtung möglich. Insbesondere bei der leitungsgebundenen Übertragung der Signale mittels elektrischer Kontakte müssen die Kupplungsteile des Hand- oder Winkelstücks, des Versorgungsgeräts und/ oder der Aufbereitungsvorrichtung miteinander gekoppelt sein.

Aber auch bei der Verwendung von Transpondern zur drahtlosen Übertragung, welche zum Senden und/ oder Empfangen von Signalen Sende- und/ oder Empfangsspulen aufweisen, ist eine Koppelung beider Kupplungsteile, auf Grund der geringen Sende-und/ oder Empfangsleistungen der Spulen, bedingt durch die geringen Baugrößen der Sende- und/ oder Empfangsspulen und der metallischen Werkstoffe in den Komponenten des medizinischen Systems, insbesondere in deren Kupplungsteilen, notwendig.

Aus der EP 1 187 375 B1 ist es des Weiteren bekannt, den menschlichen Körper als Signalpfad zur Übertragung elektrischer Signale und Daten zu verwenden.

Hierzu ist eine Sendevorrichtung in einem Kleidungsstück integriert, so dass diese an einem menschlichen Körper getragen werden kann. Eine separate Empfangseinheit ist so ausgelegt, dass diese an ein Equipment, insbesondere an einen Computer, angeschlossen werden kann, welches Daten nutzt, die von der Sendevorrichtung übertragen werden. Als nachteilig erweist sich hierbei die als separates Bauteil ausgebildete Empfangseinheit. Elektrische Signale und Daten können nicht direkt zwischen den Komponenten des medizinischen Systems, wie zum Beispiel zwischen dem Kleidungsstück und dem Computer, übertagen werden. Eine zusätzliche Übertragung der Signale und Daten von der Empfangseinheit zu dem damit verbunden Equipment ist erforderlich. In der angeführten Patentschrift wird vorgeschlagen die Empfangseinheit mittels einer USB Schnittstelle mit dem Computer zu verbinden.

DE 10 2012 105365 A1 offenbart ein System für die Überwachung der Einhaltung der Hygienevorschriften, mit dem die Einhaltung vorgegebener Hygienevorschriften beurteilt werden kann. Dieses System weist ein Sender leistungsschwacher Signale auf, die - durch kapazitive Kopplung - als Verschiebungsströme in den Körper und aus dem Körper des Nutzers fließen. Der Körper eines Nutzers fungiert als ein leitfähiges Medium. Das durch den Nutzer getragene ID-Geber verhält sich wie ein Empfänger, der kapazitiv mit dem Körper eines Nutzers gekoppelt ist und auf die Verschiebungsströme reagiert, die aus dem Körper eines Nutzers zu ihm geleitet werden, um die Signale zu detektieren. Elektroden können zur Kopplung von elektronischen Bauelementen an den menschlichen Körper verwendet werden. Die Elektroden können in Gegenstände eingearbeitet werden, die sich normalerweise in Kontakt mit dem menschlichen Körper befinden, wie zum Beispiel Armbanduhren, Kleidung oder Schuhe. Das Steuerungsmittel der Hygienevorrichtung (zum Beispiel eines Spenders von Desinfektionsmittel) wird mit einem kapazitiven Kopplungsmittel versehen, um eine elektrische Kopplung zwischen dem Körper eines Nutzers und der Steuereinheit der Vorrichtung herzustellen, um Signale von dem Vorrichtungssteuerungsmittel über den Körper eines Nutzers zu dem ID-Geber zu leiten.

Der vorliegenden Erfindung liegt daher die Aufgabe zugrunde ein medizinisches, insbesondere zahnärztliches, System zu schaffen, das es ermöglicht eine sichere Übertragung von elektrischen Signalen und/ oder Daten zwischen einer Sendeeinheit und einer Empfangseinheit zu gewährleisten. Insbesondere liegt der Erfindung die Aufgabe zugrunde auch dann eine sichere Übertragung von elektrischen Signalen zu gewährleisten, wenn die Komponenten des medizinischen Systems nicht unmittelbar miteinander gekoppelt sind.

Zur Lösung dieser Aufgabe wird ein medizinisches, insbesondere zahnärztliches, System nach Anspruch 1 vorgeschlagen.

Gemäß einem bevorzugten Ausführungsbeispiel weist die zumindest eine Sendeeinheit und die zumindest eine Empfangseinheit zum Erzeugen und/ oder Empfangen von elektromagnetischen Feldern, welche kapazitiv in den menschlichen Körper ein- bzw. auskoppelbar sind, jeweils zumindest eine elektrische Leiterplatte auf. Diese besteht vorzugsweise aus einem isolierten Basiskörper mit zumindest einer elektrischen Leiterbahn. Der Basiskörper ist hierbei bevorzugt durch ein flexibles Trägermaterial, vorzugsweise durch eine Trägerfolie, gebildet.

Die kapazitive Ein- und/ oder Auskoppelung der elektrischen Signale und/ oder Daten, insbesondere der elektromagnetischen Felder, in den menschlichen Körper des Anwenders und/ oder der zu behandelnden Person erfolgt bevorzugt durch die Kontaktierung des menschlichen Körpers mit der elektrischen Leiterplatte. Gemäß einem weiteren bevorzugten Ausführungsbeispiel erfolgt die Ein- und/ oder Auskopplung der elektromagnetischen Felder in den menschlichen Körper berührungsfrei.

Zur Versorgung der elektrischen Leiterplatte mit elektrischer Energie und zum Ausgeben von elektrischen Signalen umfasst die zumindest eine Sendeeinheit und/ oder die zumindest eine Empfangseinheit bevorzugt zumindest zwei elektrische Kontakte oder zumindest einen Energiespeicher für elektrische Energie und zumindest eine Speichereinheit zum Speichern und/ oder Ausgeben von Daten.

Des Weiteren ist die zumindest eine Sendeeinheit und/ oder die zumindest eine Empfangseinheit jeweils in einem Gehäuse angeordnet, wobei das Gehäuse derart beschaffen ist, dass die Funktionsfähigkeit der in dem Gehäuse angeordneten Sende-oder Empfangseinheit auch nach mehrmaliger Reinigung, Pflege und/ oder Sterilisation aufrechterhaltbar ist.

Das Gehäuse ist hierbei bevorzugt durch ein magnetisch und elektrisch nicht leitendes Material, insbesondere Kunststoff, Glas oder Keramik gebildet und umfasst eine Haltevorrichtung, insbesondere ein Gewinde, eine Klammer und/ oder ein Schiebeelement, so dass die zumindest eine Sende- oder Empfangseinheit lösbar von dem medizinischen System ist.

Die zumindest eine Empfangseinheit ist in oder an einem medizinischen, insbesondere zahnärztlichen, Hand- oder Winkelstück angeordnet, welches zum Antreiben eines auf eine Behandlungsstelle einwirkenden Werkzeugs und/ oder zum Abgeben von Strahlung, Fluiden, Energie und/ oder von abrasiven Medien und/ oder zur Bereitstellung von Messdaten ausgebildet ist. Die zumindest eine Sendeeinheit ist in oder an zumindest einem für den Betrieb des Hand- oder Winkelstücks ausgebildeten Zubehör angeordnet, um Daten zwischen dem Hand- oder Winkelstück und dem Zubehör zu übertragen.

Hierbei ist die zumindest eine Empfangseinheit bevorzugt in oder an einem Griff- und/ oder Haltestück des Hand- oder Winkelstücks angeordnet. Alternativ ist die zumindest eine Empfangseinheit vorzugsweise in oder an einem Betätigungs- und/ oder Bedienelement des Hand- oder Winkelstücks positioniert, so dass die Empfangseinheit kapazitiv mit einem Anwender und/ oder einer zu behandelten Person koppelbar ist.

Gemäß einem Verfahren zur Übertragung von elektrischen Signalen und/ oder Daten zwischen zumindest einer Sendeeinheit und zumindest einer Empfangseinheit eines medizinischen, insbesondere zahnärztlichen, Systems erfolgt die Übertragung der elektrischen Signale und/ oder Daten durch das zur Verfügung Stellen zumindest einer Sendeeinheit und zumindest einer Empfangseinheit, welche ausgebildet sind elektrische Signale in einen menschlichen Körper kapazitiv ein- und/ oder auszukoppeln, kapazitives Koppeln der zumindest zwei Einheiten mit dem menschlichen Körper des Anwenders und/ oder der zu behandelten Person und Herstellen eines elektrischen Pfades zwischen der zumindest einen Sendeeinheit und der zumindest einen Empfangseinheit, der sich durch den menschlichen Körper des Anwenders und/ oder der zu behandelten Person erstreckt, und übertragen der elektrischen Signale zwischen den zumindest zwei Einheiten.

Das vorliegende medizinische, insbesondere zahnärztliche, System zeichnet sich durch eine Reihe erheblicher Vorteile aus.

Durch das medizinische, insbesondere zahnärztliche, System wird es insbesondere ermöglicht Signale und/ oder Daten zwischen der zumindest einen Sendeeinheit und der zumindest einen Empfangseinheit zu übertragen, ohne dass diese unmittelbar miteinander verbunden bzw. gekoppelt werden müssen. So sind Signale und/ oder Daten mittels eines elektrischen Pfades, der sich durch den menschlichen Körper des Anwenders und/ oder der zu behandelten Person erstreckt, zwischen voneinander beabstandeten Komponenten des medizinischen, insbesondere zahnärztlichen, Systems, insbesondere dem Hand- oder Winkelstück, der Antriebs- und/ oder Steuereinheit, der Aufbereitungsvorrichtung und/ oder dem Zubehör für die mehreren Komponenten, übertragbar.

Des Weiteren wird durch das medizinische, insbesondere zahnärztliche, System auch dann eine sichere Übertragung von Signalen und/ oder Daten zwischen der zumindest einen Sendeeinheit und der zumindest einen Empfangseinheit gewährleistet, wenn die zumindest zwei Einheiten des medizinischen Systems nicht unmittelbar miteinander gekoppelt sind. Die Nutzung des menschlichen Körpers des Anwenders und/ oder der zu behandelten Person als elektrischen Pfad zur Übertragung der elektrischen Signale und/ oder Daten verhindert die Gefahr einer Gefährdung des Signal- und/ oder Datentransfers. Dieser kann bei einer induktiven Energie- und/ oder Datenübertragung, wie aus dem Stand der Technik bekannt, durch elektrische Gegenfelder, welche durch metallische Bauteile im Bereich der Sende- und Empfangsspulen erzeugt werden, gestört werden.

Nachfolgend wird die Erfindung anhand mehrerer Ausführungsbeispiele und in Verbindung mit den beigefügten Zeichnungen erläutert.

Dabei zeigt:
Figur 1 ein erstes Ausführungsbeispiel des medizinischen, insbesondere zahnärztlichen, Systems mit einer Antriebs- und/ oder Steuereinheit, einem medizinischen, insbesondere zahnärztlichen, Handstück sowie einem Zubehör für den Betrieb des Handstücks, welches in diesem Ausführungsbeispiel als Brille, insbesondere als Filterbrille zur Erkennung einer Anomalie an einem Zahn ausgebildet ist,
Figur 1A einen Ausschnitt aus dem medizinischen Handstück aus Figur 1,
Figur 1B einen Ausschnitt aus dem Zubehör für den Betrieb des Handstücks, insbesondere aus der Brille aus Figur 1,
Figur 2 ein zweites Ausführungsbeispiel des medizinischen, insbesondere zahnärztlichen, Systems mit einem medizinischen Handstück, einer Antriebs- und/ oder Steuereinheit, sowie mit mehreren Ausführungsbeispielen des für den Betrieb des Handstücks ausgebildeten Zubehörs,
Figur 3 ein drittes Ausführungsbeispiel des medizinischen, insbesondere zahnärztlichen, Systems mit einem medizinischen, insbesondere zahnärztlichen Winkelstück und einer daran anschließbaren, insbesondere kabellosen, Antriebs und/ oder Steuereinheit, einer Basiseinheit, sowie einer zweiten, insbesondere in einer Behandlungseinheit integrierten, Antriebs- und/ oder Steuereinheit,
Figur 4 ein viertes Ausführungsbeispiel des medizinischen, insbesondere zahnärztlichen, Systems mit einer Aufbereitungsvorrichtung zur Aufbereitung eines medizinischen, insbesondere zahnärztlichen, Hand- oder Winkelstücks und einem für den Betrieb der Aufbereitungsvorrichtung ausgebildeten Zubehör;

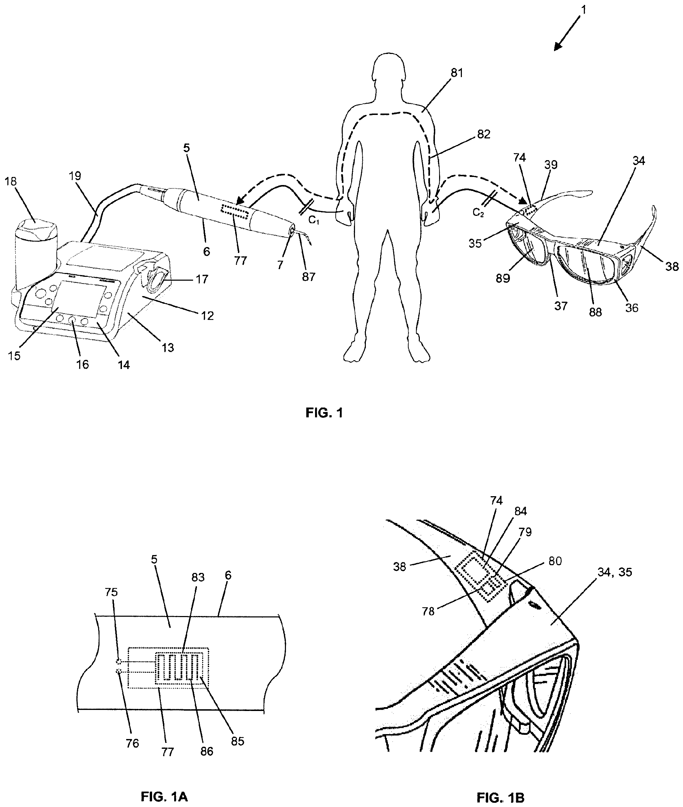

Das in Figur 1 dargestellte erste Ausführungsbeispiel des medizinischen, insbesondere zahnärztlichen, Systems 1 umfasst eine Antriebs- und/ oder Steuereinheit 12 mit einem medizinischen, insbesondere zahnärztlichen, Handstück 5 und ein für den Betrieb des Handstücks 5 ausgebildetes Zubehör 34. Das Zubehör ist hierbei als Brille 34, insbesondere als Filterbrille zur Erkennung einer Anomalie an einem Zahn ausgebildet.

Die Antriebs- und/ oder Steuereinheit 12 umfasst ein Gehäuse 13 mit einer Handstückaufnahme 17. An der Frontseite weist die Antriebs- und/ oder Steuereinheit 12 eine Bedieneinheit 14 auf. Diese ist durch eine Anzeige 15 mit mehreren Betätigungselementen 16 zur Anzeige und Einstellung der Betriebsparameter der Antriebs- und/ oder Steuereinheit 12 und/ oder des medizinischen Handstücks 5 gebildet. Des Weiteren umfasst die Antriebs- und/ oder Steuereinheit 12 einen Fluidtank 18. Um schließlich das medizinische Handstück 5 anzutreiben, anzusteuern und/ oder mit Sprayluft und/ oder Spraywasser zur Kühlung oder mit elektrischer Energie zu versorgen, ist dieses mittels der Versorgungsleitung 19 mit der Antriebs- und/ oder Steuereinheit 12 verbunden.

Das Handstück 5 selbst ist durch ein Griffstück 6 gebildet. In dem Griffstück 6 ist vorzugsweise ein Antrieb zum Antreiben des Werkzeugs 87 angeordnet. Neben dem Antrieb weist das Handstück 5 eine Lichtquelle 7 auf. Diese ist bevorzugt ringförmig ausgebildet und umfasst zur Beleuchtung der Behandlungsstelle zumindest eine erste Leuchtdiode und zur Erkennung einer Anomalie an einer Behandlungsstelle zumindest eine zweite Leuchtdiode. Die zumindest eine zweite Leuchtdiode emittiert bevorzugt elektromagnetische Strahlung, welche eine oder mehrere Wellenlängen im Bereich von etwa 380 nm bis 420 nm umfasst. Die zumindest zwei Leuchtdioden sind wahlweise von der Antriebs- und/ oder Steuereinheit 12 ansteuerbar, insbesondere aktivierbar, so dass die Lichtquelle 7 entweder zur Beleuchtung der Behandlungsstelle oder zur Erkennung einer Anomalie dient.

Das als Brille 34, insbesondere als Filterbrille, ausgebildete Zubehör umfasst zwei Filter 88, 99, die ausgebildet sind, von dem zu untersuchenden Zahn abgestrahlte Strahlung durchzulassen, deren Wellenlänge zum Nachweis der Anomalie an einem Zahn geeignet ist. Die Filter 88, 89 sind hierbei in einer Aufnahme 36 des Brillenrahmens 35 der Filterbrille 34 aufgenommen. Neben der Aufnahme 36 umfasst die Brille 34 zwei Bügel 38, 39 und eine Auflage 37 zur Befestigung der Filter 88, 89 an einem Anwender.

Zur automatischen Auswahl der zumindest einen ersten Leuchtdiode der Lichtquelle 7 zur Beleuchtung der Behandlungsstelle oder der zumindest einen zweiten Leuchtdiode, welche in Verbindung mit der Filterbrille 34 zur Erkennung der Anomalie an einem Zahn dient, weist das medizinische System 1 eine erste Sende- und/ oder Empfangseinheit 74 und eine zweite Sende- und/ oder Empfangseinheit 77 auf. Die erste Sende- und/ oder Empfangseinheit 74 ist hierbei in der Filterbrille 34, insbesondere in dem Bügel 39 oder in der Aufnahme 37, und die zweite Sende- und/ oder Empfangseinheit 77 in dem Griffstück 6 des Handstücks 5 angeordnet. Beide Einheiten, sowohl die erste als auch zweite Sende- und/ oder Empfangseinheit 74, 77, sind zur kapazitiven Ein- und/ oder Auskoppelung von elektrischen Signalen C₁, C₂ in den menschlichen Körper 81 ausgebildet. Werden somit beide Einheiten 74, 77, insbesondere die Filterbrille 34 und das Handstück 5, mit dem menschlichen Körper 81 des Anwenders gekoppelt, ist ein elektrischer Pfad 82 zur Übertragung von elektrischen Signalen zwischen der ersten Sende- und/ oder Empfangseinheit 74 und der zweiten Sende- und/ oder Empfangseinheit 77 herstellbar. Von der in dem Handstück 5 angeordneten zweiten Sende- und/ oder Empfangseinheit 77 empfangene Signale werden bevorzugt mittels der Versorgungsleitung 19 an die Antriebs- und/ oder Steuereinheit 12, insbesondere an deren Steuerschaltung, weitergeleitet. Empfängt somit die Antriebs- und/ oder Steuereinheit 12 zumindest ein elektrisches Signal von der ersten Sende- und/ oder Empfangseinheit 74 der Filterbrille 34 (der Anwender trägt die Filterbrille 34 zur Behandlung), aktiviert diese automatisch die zumindest eine zweite Leuchtdiode zur Erkennung der Anomalie an einem Zahn. Wird kein Signal empfangen (der Anwender hat die Filterbrille 34 abgelegt), aktiviert die Antriebs- und/ oder Steuereinheit 12 automatisch die zumindest eine erste Leuchtdiode zur Beleuchtung der Behandlungsstelle. Ein manuelles umschalten zwischen der zumindest einen ersten und der zumindest einen zweiten Leuchtdiode ist hierdurch nicht mehr notwendig.

In Figur 1A ist ein Ausschnitt aus dem medizinischen Handstück 5 aus Figur 1 mit der zweiten Sende- und/ oder Empfangseinheit 77 abgebildet. Die in dem Griffstück 6 angeordnete Sende- und/ oder Empfangseinheit 77 ist bevorzugt durch eine elektrische Leiterplatte 83 zum Erzeugen und/ oder Empfangen von elektromagnetischen Feldern gebildet. Die elektrische Leiterplatte 83 selbst weist vorzugsweise einen isolierten Basiskörper 85 und zumindest eine elektrische Leiterbahn 86 auf. Der Basiskörper 85 ist hierbei bevorzugt durch eine flexibles Trägermaterial, insbesondere durch eine Trägerfolie, gebildet. Um die elektrische Leiterplatte 83 mit elektrischer Energie zu versorgen und/ oder empfangene und/ oder gesendete elektrische Signale auszugeben, weist die die Sende- und/ oder Empfangseinheit 77 zumindest zwei elektrische Kontakte 75, 76 auf. Des Weiteren ist die elektrische Leiterplatte 83 bevorzugt derart in dem Griffstück 6 angeordnet, dass diese kapazitiv mit einem Anwender und/ oder einer zu behandelten Person koppelbar ist. Hierzu ist diese vorzugsweise an der Innen- oder Außenseite des Griffstücks 6 angeordnet. Das Griffstück 6 selbst ist hierbei bevorzugt aus einem magnetisch und elektrisch nicht leitenden Material, insbesondere Kunststoff, Glas oder Keramik, gefertigt.

In Figur 1B ist ein Ausschnitt aus der Filterbrille 34 aus Figur 1 abgebildet. In dem Brillenrahmen 35 der Filterbrille 34, insbesondere in dem Bügel 38, ist die erste Sende-und/ oder Empfangseinheit 74 angeordnet, so dass diese mit dem Anwender kapazitiv koppelbar ist. Die erste Sende- und/ oder Empfangseinheit 74 umfasst hierbei, ebenso wie die zweite Sende- und/ oder Empfangseinheit 77, eine elektrische Leiterplatte 84 zum Erzeugen und/ oder Empfangen von elektromagnetischen Feldern. Um die Leiterplatte 84 mit elektrischer Energie zu versorgen weist die erste Sende- und/ oder Empfangseinheit 74 zumindest einen Energiespeicher 78 auf. Eine Speichereinheit 79 dient zum Speichern und/ oder Ausgeben von elektrischen Signalen und/ oder Daten. Die Sende- und/ oder Empfangseinheit 74 ist bevorzugt in einem Gehäuse 80 angeordnet, wobei das Gehäuse 80 derart beschaffen ist, dass die Funktionsfähigkeit der Sende- und/ oder Empfangseinheit 74 auch nach mehrmaliger Reinigung, Pflege und/ oder Sterilisation aufrechterhaltbar ist. Hierbei ist die Leiterplatte 84, der Energiespeicher 78 und die Speichereinheit 79 bevorzugt mittels eines Spritzgussverfahrens in einem Kunststoffgehäuse 80 eingehaust. Alternativ kann das Gehäuse 80 aus Glas oder Keramik bestehen.

Um die erste Sende- und/ oder Empfangseinheit 74 lösbar mit dem medizinischen System, insbesondere mit der Filterbrille 34 zu verbinden, weist das Gehäuse 80 der ersten Sende- und/ oder Empfangseinheit 74 in einem alternativen Ausführungsbeispiel eine Haltevorrichtung, insbesondere ein Gewinde, eine Klammer und/ oder ein Schiebeelement, auf.

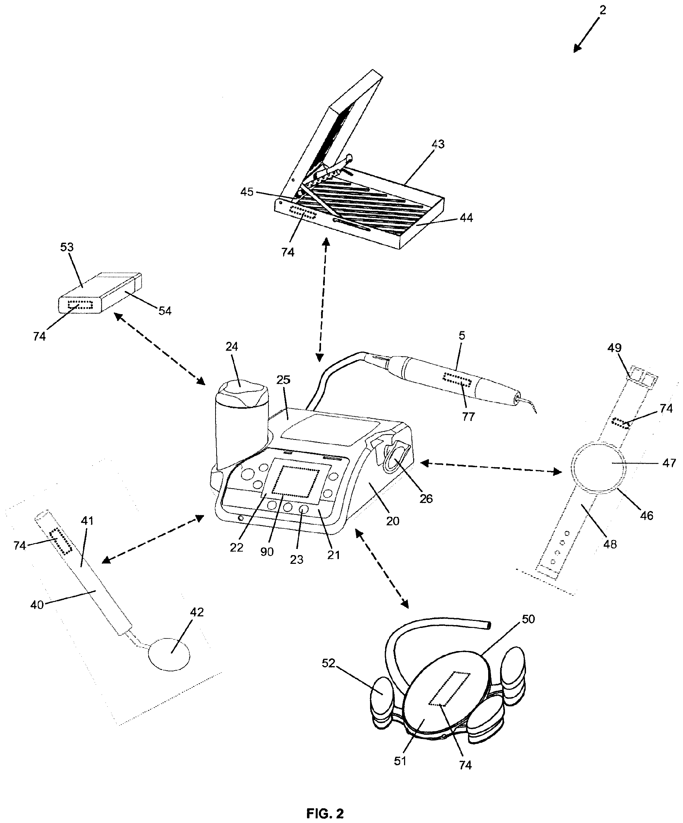

Figur 2 zeigt ein zweites Ausführungsbeispiel des medizinischen, insbesondere zahnärztlichen, Systems 2 mit zumindest einem medizinischen, insbesondere zahnärztlichen, Handstück 5 und einer Antriebs- und/ oder Steuereinheit 20. Des Weiteren sind mehrere Ausführungsbeispiele für das für den Betrieb des Handstücks 5 und/ oder für den Betrieb der Antriebs- und/ oder Steuereinheit 20 ausgebildete Zubehör abgebildet. Das Zubehör ist hierbei bevorzugt als Handspiegel 40, Instrumententräger 43, Anzeigeeinheit 46, Fußanlasser 50 und als Identifizierungselement 53 zur Benutzererkennung ausgebildet und weist jeweils eine erste Sende- und/ oder Empfangseinheit 74 auf.

Die Antriebs- und/ oder Steuereinheit 20 umfasst ein Gehäuse 25 mit einer Handstückaufnahme 26 und einem Fluidtank 24. An der Rückseite ist über einen Versorgungsschlauch das zumindest eine medizinische Handstück 5 mit der zweiten Sende- und/ oder Empfangseinheit aus Figur 1 anschließbar. An der Frontseite weist die Antriebs- und/ oder Steuereinheit 20 eine zweite Ausführungsform der Bedieneinheit 21 auf. Neben einer Anzeige 22 und zumindest einem Betätigungselement 23 umfasst die Einheit 21 eine dritte Sende- und/ oder Empfangseinheit 90 zum kapazitiven Ein-und/ oder Auskoppelung von elektrischen Signalen in den menschlichen Körper 81. Die Sende- und/ oder Empfangseinheit 90 ist hierbei bevorzugt in der Anzeige 22 aufgenommen, welche wiederum bevorzugt als Berührungsbildschirm ausgebildet ist.

Sowohl die zweite Sende- und/ oder Empfangseinheit 77 des Handstücks 5, als auch die dritte Sende- und/ oder Empfangseinheit 90 der Antriebs- und/ oder Steuereinheit 20 sind mit der Steuerschaltung der Einheit 20 verbunden.

Das erste Ausführungsbeispiel des Zubehörs ist als Handspiegel 40 mit einem Griffstück 41 und einem daran befestigten Spiegelelement 42 ausgebildet. Die erste Sende- und/ oder Empfangseinheit 74 ist hierbei bevorzugt in dem Griffstück 41 angeordnet. Werden der Handspiegel 40 und das Handstück 5 und/ oder die Antriebs-und/ oder Steuereinheit 20 mit dem menschlichen Körper 81 des Anwenders gekoppelt, entsteht eine elektrischer Pfad und elektrische Signale können zwischen der ersten Sende- und/ oder Empfangseinheit 74 und der zweiten und/ oder dritten Sende- und/ oder Empfangseinheit 77, 90 übertragen werden. Empfängt somit beispielsweise die Sende- und/ oder Empfangseinheit 90 in der Antriebs- und/ oder Steuereinheit 20 oder die Sende- und/ oder Empfangseinheit 77 in dem Handstück 5 zumindest ein elektrisches Signal von der Sende- und/ oder Empfangseinheit 74 des Handspiegels 40, aktiviert die Steuerschaltung der Einheit 20 bevorzugt automatisch das zumindest eine Hand- oder Winkelstück 5, insbesondere dessen Antrieb. Hierdurch wird eine automatische Start- und Stopp-Funktion des Hand- oder Winkelstücks 5 ermöglicht. Ein manuelles Betätigen des Hand- oder Winkelstücks 5, vorzugsweise mittels eines An-oder Ausschalters ist nicht notwendig. Des Weiteren wird der Antriebs- und/ oder Steuereinheit 20 an Hand der Übertragung der elektrischen Signale und/ oder Daten angezeigt, dass ein Anwender präsent ist, wodurch weitere Funktionen, wie zum Beispiel der Wechsel der Antriebs- und/ oder Steuereinheit 20 von einem Ruhezustand in einen aktiven Modus, ausbildbar sind.

Das zweite Ausführungsbeispiel des Zubehörs ist als Instrumententräger 43 ausgebildet. Dieser umfasst neben einem Gehäuse 44 zumindest eine Anschlussvorrichtung 45 mit zumindest einem Kupplungselement zur Aufnahme des zumindest einen medizinischen Hand- oder Winkelstücks 5 und/ oder eines Werkzeugs. Die Anschlussvorrichtung 45 ist vorzugsweise schwenkbar in dem Gehäuse 44 gelagert. Die erste Sende- und/ oder Empfangseinheit 74 ist hierbei bevorzugt in dem Gehäuse 44 des Instrumententrägers 43 angeordnet. Elektrische Signale und/ oder Daten, vorzugsweise Betriebsparameter des zumindest einen in dem Instrumententräger 43 aufgenommenen Hand- oder Winkelstücks 5 oder des Werkzeugs, können hierdurch zwischen der ersten Sende- und/ oder Empfangseinheit 74 des Instrumententrägers 43 mittels des menschlichen Körpers des Anwenders und der mit dem Körper gekoppelten zweiten Sende- und/ oder Empfangseinheit 77 des Handstücks 5 oder der dritten Sende- und/ oder Empfangseinheit 90 der Antriebs- und/ oder Steuereinheit 20 übertragen werden. Empfängt die zweite und/ oder dritte Sende-und/ oder Empfangseinheit 77, 90 in der Antriebs- und/ oder Steuereinheit 20 oder in dem Handstück 5 elektrische Signale und/ oder Daten, insbesondere die Betriebsparameter, von der ersten Sende- und/ oder Empfangseinheit 74 des Instrumententrägers 43, stellt die Antriebs- und/ oder Steuereinheit 20, insbesondere deren Steuerschaltung, automatisch die Betriebsparameter für das zumindest eine dem Instrumententräger 43 zugeordnete Hand- oder Winkelstück 5 und/ oder Werkzeug ein.

Ein weiteres Ausführungsbeispiel des Zubehörs ist als Anzeigeeinheit 46, welche bevorzugt die Form einer Armbanduhr aufweist, ausgebildet. Neben einer Anzeige 47 weist die Anzeigeeinheit 46 ein Armband 48 mit einem Verschlusselement 49 auf, um bevorzugt mit einem Arm eines Anwenders verbindbar zu sein. Die erste Sende- und/ oder Empfangseinheit 74 ist hierbei bevorzugt in dem Armband 48 angeordnet und mit der Anzeige 47 elektrisch verbunden. Elektrische Signale und/ oder Daten, vorzugsweise erfasste und/ oder eingestellte Paramater des Hand- oder Winkelstücks 5 und/ oder der Antriebs- und/ oder Steuereinheit 20, können somit von der Sende- und/ oder Empfangseinheit 77 des Handstücks 5 oder der Sende- und/ oder Empfangseinheit 90 der Antriebs- und/ oder Steuereinheit 20 mittels des menschlichen Körpers des Anwenders zu der Sende- und/ oder Empfangseinheit 74 und der Anzeige 47 der Anzeigeeinheit 46 übertragen werden. Dies ermöglicht erfasste und/ oder eingestellte Parameter des Hand- oder Winkelstücks 5 und/ oder der Antriebs- und/ oder Steuereinheit 20 direkt im Behandlungsumfeld des Anwenders anzuzeigen.

Das vierte Ausführungsbeispiel des Zubehörs ist als Fußanlasser 50 für die Antriebs-und/ oder Steuereinheit 20 ausgebildet. Der Fußanlasser 50 dient hierbei bevorzugt zur Aktivierung eines elektrischen Antriebs in dem Handstück 5, welcher von der Steuerschaltung in der Antriebs- und/ oder Steuereinheit 20 angesteuert wird. Der Fußanlasser 50 weist zumindest ein Pedal 51 sowie zumindest ein Betätigungselement 52 auf. Das Pedal 51 dient bevorzugt zum Regeln variabler Parameter, wie zum Beispiel verschiedener Drehzahlen, und das Betätigungselement 52 zum Ein- und/ der Ausschalten des Fußanlassers 50 und/ oder zum Umschalten zwischen den verschiedenen variablen Parametern. Beide Elemente, das Pedal 51 und das Betätigungselement 52 senden bei einer Betätigung elektrische Signale an eine Steuereinheit in dem Fußanlasser 50, welche wiederum mit der ersten Sende- und/ oder Empfangseinheit 74 verbunden ist. Die Sende- und/ oder Empfangseinheit 74 ist bevorzugt in dem Pedal 51 angeordnet. Alternativ kann diese auch in dem zumindest einen Betätigungselement 52 positioniert sein. Elektrische Signale und/ oder Daten, wie zum Beispiel die Drehzahlwerte, können hierdurch von der Sende- und/ oder Empfangseinheit 74 des Fußanlassers 50, mittels des menschlichen Körpers des Anwenders zu der zweiten Sende- und/ oder Empfangseinheit 77 in dem medizinischen Handstück 5 und/ oder zu der dritten Sende- und/ oder Empfangseinheit 90 in der Antriebs- und/ oder Steuereinheit 20 übertragen werden. Die Steuerschaltung der Antriebs- und/ oder Steuereinheit 20 aktiviert somit, abhängig von den empfangenen Signalen und/ oder Daten von dem Fußanlasser 50, den elektrischen Antrieb in dem Handstück 5. Des Weiteren kann die erste Sende- und/ oder Empfangseinheit 74 des Fußanlassers 50 auch elektrische Signale und/ oder Daten empfangen, so dass der zumindest eine Fußanlasser 51 mehreren Antriebs- und/ oder Steuereinheiten zuweisbar ist.

Das fünfte Ausführungsbeispiel des Zubehörs ist als Identifizierungselement 53 ausgebildet. Das Element 53 besteht aus einem Gehäuse 54, in welchem die erste Sende- und/ oder Empfangseinheit 74 angeordnet ist. Das Identifizierungselement 53 dient hierbei zur Erkennung des Anwenders des medizinischen Systems 2, insbesondere zur automatischen Auswahl von Betriebsprogrammen und/ oder Betriebsparametern in der Antriebs- und/ oder Steuereinheit 20. Elektrische Signale und/ oder Daten, wie zum Beispiel Anwendercodes, können von der ersten Sende- und/ oder Empfangseinheit 74 des Identifizierungselements 53, mittels des menschlichen Körpers des Anwenders zu der zweiten Sende- und/ oder Empfangseinheit 77 in dem medizinischen Handstück 5 und/ oder zu der dritten Sende- und/ oder Empfangseinheit 90 der Antriebs- und/ oder Steuereinheit 20 übertragen werden. Die Antriebs- und/ oder Steuereinheit 20 stellt so, abhängig von den empfangenen Signalen und/ oder Daten die Betriebsprogramme und/ oder Betriebsparamater automatisch ein.

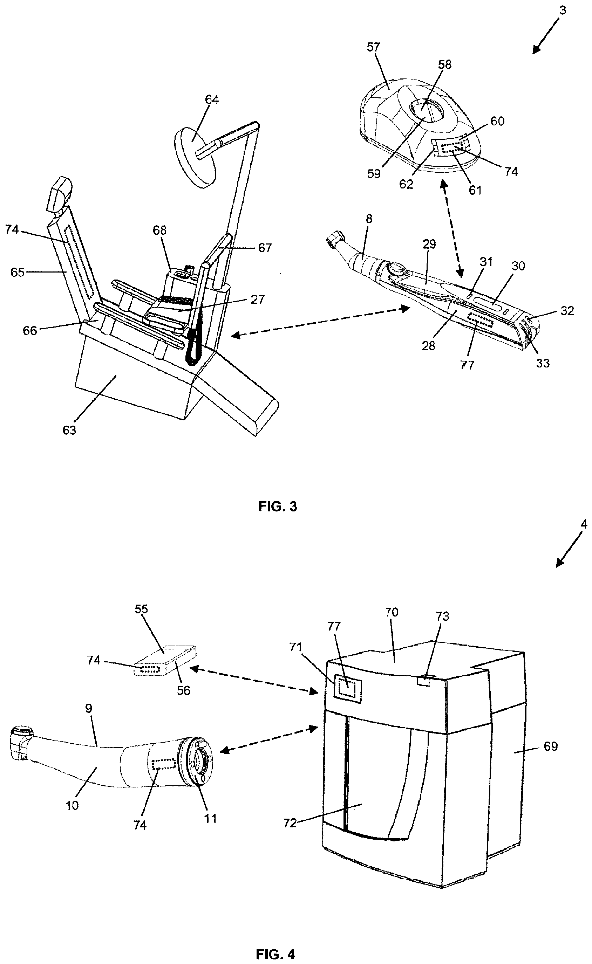

In Figur 3 ist ein drittes Ausführungsbeispiel des medizinischen, insbesondere zahnärztlichen, Systems 3 mit einem medizinischen, insbesondere zahnärztlichen, Winkelstück 8 und einer daran anschließbaren ersten, insbesondere kabellosen, Antriebs und/ oder Steuereinheit 28, einer Basiseinheit 57, sowie einer zweiten, insbesondere in bzw. an einer Behandlungseinheit 63 angeordneten, Antriebs- und/ oder Steuereinheit 27 abgebildet.

Die an das Winkelstück 8 anschließbare, insbesondere kabellose, Antriebs- und/ oder Steuereinheit 28 weist ein Gehäuse 29 auf, in welchem eine Steuerschaltung zum Ansteuern eines in der Antriebs- und/ oder Steuereinheit 28 aufgenommenen Antriebs, insbesondere eines Elektromotors, angeordnet ist. Zusätzlich zu dem Antrieb weist die Einheit 28 bevorzugt zumindest einen Energiespeicher zur Versorgung des Antriebs und/ oder der Steuerschaltung mit elektrischer Energie sowie zumindest einen Datenspeicher zum Speichern von Daten auf. An der Rückseite des Gehäuses 29 weist die Antriebs- und/ oder Steuereinheit 28 zumindest zwei Ladekontakte 32, 33 für den Energiespeicher auf. Zur Anzeige und Einstellung von erfassten und/ oder vorgegebenen Betriebsparametern und/ oder Betriebsprogrammen des in der Antriebs-und/ oder Steuereinheit 28 angeordneten Antriebs und/ oder des anschließbaren Winkelstücks 8 weist die Antriebs- und/ oder Steuereinheit 28 eine Anzeige 30 sowie zumindest ein Betätigungselement 31 auf.

Um von einer ersten Sende- und/ oder Empfangseinheit 74, welche bevorzugt in der Basisstation 57 oder in der Antriebs- und/ oder Steuereinheit 27 der Behandlungseinheit 63 angeordnet ist, vorgegebene Betriebsprogramme und/ oder Betriebsparameter zu empfangen, weist die Antriebs- und/ oder Steuereinheit 28 eine zweite Sende- und/ oder Empfangseinheit 77 auf. Die zweite Sende- und/ oder Empfangseinheit 77 ist hierbei zum Empfangen und Senden von Signalen und/ oder Daten ausgebildet, um gemessene Betriebsparameter des Antriebs und/ oder des Winkelstücks 8 an die Basisstation 57 und/ oder an die Behandlungseinheit 63 zu übertragen. Beide Einheiten, die erste Sende- und/ oder Empfangseinheit 74 und die zweite Sende- und/ oder Empfangseinheit 77, sind auch hier zur kapazitiven Ein- und/ oder Auskoppelung von elektrischen Signalen in den menschlichen Körper ausgebildet. Werden somit beispielsweise die Antriebs- und/ oder Steuereinheit 28, die Basiseinheit 57 und/ oder die Behandlungseinheit 63 mit dem menschlichen Körper 81 des Anwenders gekoppelt, können elektrische Signale und/ oder Daten zwischen der ersten Sende- und/ oder Empfangseinheit 74 und der zweiten Sende- und/ oder Empfangseinheit 77 übertragen werden.

Die erste Sende- und/ oder Empfangseinheit 74 der Basisstation 57, welche eine Aufnahme 58 für die Antriebs- und/ oder Steuereinheit 28 sowie eine Ladeeinheit 59 für diese aufweist, ist hierzu bevorzugt in oder an einer Anzeige 61 einer Bedieneinheit 60 mit zumindest einem Betätigungselement 62 der Basisstation 57 angeordnet und mit einer Speichereinheit verbunden, welche in der Basisstation 57 angeordnet ist und auf welcher Betriebsprogramme und/ oder Betriebsprogramme speicherbar sind. Die Anzeige 61 ist auch bei diesem Ausführungsbeispiel bevorzugt als Berührungsbildschirm ausgebildet. Elektrische Signale und/ oder Daten, insbesondere die Betriebsparameter und/ oder Betriebsprogramme können hierdurch während der Auswahl durch den Anwender mittels des Berührungsbildschirms 60 von der Speichereinheit der Basiseinheit 57 auf die Antriebs- und/ oder Steuereinheit 28 übertragen werden. Gleichzeitig können von der Antriebs- und/ oder Steuereinheit 28 erfasste Betriebsparameter auf der Speichereinheit der Basiseinheit 57 abgelegt werden.

Die Sende- und/ oder Empfangseinheit 74 der zweiten Antriebs- und/ oder Steuereinheit 27 ist bevorzugt in oder an der Lehne 65 bzw. in oder an der Sitz- und/ oder Liegefläche 66 für die zu behandelnde Person der Behandlungseinheit 63 angeordnet und mit der zweiten Antriebs- und/ oder Steuereinheit 27 verbunden. Neben der zweiten Antriebs-und/ oder Steuereinheit 27 und der Sitz- und/ oder Liegefläche 66 weist die Behandlungseinheit 63 eine Beleuchtung 64, ein Spülbecken 68 sowie eine Versorgungsleitung 67 auf. Die Versorgungsleitung 67 dient u.a. zur Versorgung der Antriebs- und/ oder Steuereinheit 27 mit elektrischer Energie. Um elektrische Signale und/ oder Daten zwischen der zweiten Steuer und/ oder Antriebseinheit 27 und der kabellosen Antriebs- und/ oder Steuereinheit 28 zu übertragen, sind die erste Sende-und/ oder Empfangseinheit 74 in der Lehne 65 und die zweite Sende- und/ oder Empfangseinheit 77 in der Antriebs- und/ oder Steuereinheit 28 zum kapazitiven Ein-und/ oder Auskoppelung von elektrischen Signalen in den menschlichen Körper ausgebildet und derart in der Lehne 65 und der Antriebs- und/ oder Steuereinheit 28 angeordnet, dass diese mit dem menschlichen Körper des Patienten koppelbar sind. Hierzu ist die zweite Sende- und/ oder Empfangseinheit 77 in der kabellosen Antriebs-und/ oder Steuereinheit 28 bevorzugt derart ausgebildet, dass die elektrischen Signale und/ oder Daten, insbesondere die erzeugten elektromagnetischen Felder, auch dann mit dem menschlichen Körper des Patienten koppelbar sind, wenn dieser die zweite Sende- und/ oder Empfangseinheit 77 nicht unmittelbar berührt bzw. kontaktiert. Eine Übertragung der elektrischen Signale und/ oder Daten erfolgt hierbei berührungsfrei. Des Weiteren sind durch die Anordnung gemäß Figur 4 elektrische Signale und/ oder Daten von der Antriebs- und/ oder Steuereinheit 27 mittels der kabellosen Antriebs-und/ oder Steuereinheit 28 auf die Basiseinheit 57 übertragbar.

Figur 4 zeigt ein viertes Ausführungsbeispiel des medizinischen, insbesondere zahnärztlichen, Systems 4 mit einer Aufbereitungsvorrichtung 69 zur Aufbereitung eines medizinischen, insbesondere zahnärztlichen, Hand- oder Winkelstücks 9 und einem für den Betrieb der Aufbereitungsvorrichtung ausgebildeten Zubehör 55. Die Aufbereitungsvorrichtung 69 ist vorzugsweise in Form eines Sterilisators, insbesondere eines Dampfsterilisators ausgebildet. Der Sterilisator umfasst hierbei ein Gehäuse 70 mit mehreren Außenwänden. Eine Außenwand, insbesondere eine Seitenwand, bildet vorzugsweise die Bedienungsseite der Aufbereitungsvorrichtung 69. Diese weist vorzugsweise zumindest eine Bedieneinheit 71 und zumindest ein Betätigungselement 73, welche insbesondere zur Auswahl unterschiedlicher Betriebsprogramme oder zur Einstellung von Betriebsparametern dienen und mit einer Steuervorrichtung zum Steuern und/ oder Regeln des Aufbereitungsprozesses verbunden sind, auf. Neben der Bedieneinheit 71 umfasst das Gehäuse 70 des Sterilisators eine Öffnung, die mit einer Aufbereitungskammer des Sterilisators verbunden ist. Durch die Öffnung kann ein aufzubereitendes Hand- oder Winkelstück 9 oder medizinisches Zubehör in die Aufbereitungskammer eingebracht oder daraus entnommen werden. Die Öffnung ist mittels einer Tür 72, welche bevorzugt drehbar über ein Scharnier zu den mehreren Wänden des Sterilisators gelagert ist, verschließbar. In der Aufbereitungskammer ist bevorzugt eine Medienzuführung zur Versorgung des zumindest einen aufgenommenen Hand- oder Winkelstücks 9 mit einem Reinigungs- und/ oder Pflegemittel vorgesehen.

Zur automatischen Auswahl unterschiedlicher Betriebsprogramme oder Betriebsparameter sowie zur Erkennung des Anwenders weist das medizinische System 4 zumindest eine erste Sende- und/ oder Empfangseinheit 74 und zumindest eine zweite Sende- und/ oder Empfangseinheit 77 auf. Die erste Sende- und/ oder Empfangseinheit 74 ist hierbei in dem zumindest einen aufzubereitenden medizinischen Winkelstück 9, insbesondere in oder an dessen Griffstück 10, und/ oder in dem für den Betrieb der Aufbereitungsvorrichtung 69 ausgebildeten Zubehör 55, insbesondere in dem Identifizierungselement 55, vorzugsweise in dessen Gehäuse 56, angeordnet. Das Winkelstück 9 weist an einem hinteren Ende bevorzugt ein Kupplungselement 11 zum Ankuppeln des Winkelstücks 9 an die Aufbereitungsvorrichtung 69, insbesondere an deren Medienzuführung, auf. Die zweite Sende- und/ oder Empfangseinheit 77 ist in diesem Ausführungsbeispiel in oder an der Bedieneinheit 71 der Aufbereitungsvorrichtung 69 angeordnet. Alternativ kann dieses in oder an dem Betätigungselement 73 oder in oder an der Tür 72 der Aufbereitungsvorrichtung 69 angeordnet sein.

Sowohl die erste Sende- und/ oder Empfangseinheit 74 als auch die zweite Sende- und/ oder Empfangseinheit 77 sind auch in diesem Ausführungsbeispiel zur kapazitiven Ein-und/ oder Auskoppelung von elektrischen Signalen in den menschlichen Körper ausgebildet. Erfolgt eine Koppelung der ersten Sende- und/ oder Empfangseinheit 74 und der zweiten Sende- und/ oder Empfangseinheit 77, insbesondere des zumindest einen aufzubereitenden Winkelstücks 9, des Identifizierungselements 55 und der Aufbereitungsvorrichtung 69, mit dem menschlichen Körper 81 des Anwenders, so entsteht ein elektrischer Pfad 82 zur Übertragung von elektrischen Signalen zwischen den zumindest zwei Sende- und/ oder Empfangseinheiten 74, 77. Elektrische Signale, die von der ersten Sende- und/ oder Empfangseinheit 74 des medizinischen Winkelstücks 9 gesendet werden, beinhalten insbesondere die Seriennummer des Winkelstücks 9 und/ oder Betriebsparameter für die Aufbereitungsvorrichtung 69. Die Sende- und/ oder Empfangseinheit 74 des Identifizierungselements 55 sendet vorzugsweise einen Anwendercode zur Erkennung des Anwenders. Die zweite Sende-und/ oder Empfangseinheit 77 empfängt die von der ersten Sende- und/ oder Empfangseinheit 74 gesendeten elektrischen Signale und leitet diese bevorzugt an die Steuervorrichtung der Aufbereitungsvorrichtung 69 weiter. Betriebsparameter und/ oder Betriebsprogramme werden auf diese Weise abhängig von den aufzubereitenden Winkelstücken 9 automatisch ausgewählt. Des Weiteren erfolgt durch das Empfangen von Anwendercodes eine Zuordnung des Anwenders der Aufbereitungsvorrichtung 69 zu den aufbereitenden Hand- oder Winkelstücken 9. Eine manuelle Eingabe der Anwenderdaten und/ oder der Betriebsparameter für das zu reinigende Winkelstück 9 ist hierdurch nicht mehr notwendig.

Die Erfindung ist nicht auf das beschriebene Ausführungsbeispiel beschränkt, sondern umfasst alle Ausführungen, die in den beiliegenden Ansprüchen definiert sind.

## Patentansprüche

1. Medizinisches, insbesondere zahnärztliches, System (1), umfassend: ein medizinisches, insbesondere zahnärztliches, Hand- oder Winkelstück (5, 8), zumindest eine Sendeeinheit (74) und zumindest eine Empfangseinheit (77), wobei die zumindest zwei Einheiten (74, 77) zur kapazitiven Ein- und/ oder Auskoppelung von elektrischen Signalen und/ oder Daten in einen menschlichen Körper (81) ausgebildet sind, so dass ein elektrischer Pfad (82) zur Übertragung von elektrischen Signalen und/ oder Daten zwischen den zumindest zwei Einheiten (74, 77), der sich durch den menschlichen Körper (81) des Anwenders und/ oder der zu behandelten Person erstreckt, herstellbar ist, **dadurch gekennzeichnet, dass**
die zumindest eine Empfangseinheit (77) in oder an dem medizinischen, insbesondere zahnärztlichen, Hand- oder Winkelstück (5, 8) angeordnet ist, welches zum Antreiben eines auf eine Behandlungsstelle einwirkenden Werkzeugs und/ oder zum Abgeben von Strahlung, Fluiden, Energie und/ oder von abrasiven Medien und/ oder zur Bereitstellung von Messdaten ausgebildet ist, und dass die zumindest eine Sendeeinheit (74) in oder an zumindest einem für den Betrieb des Hand- oder Winkelstücks (5, 8) ausgebildeten Zubehör (34) des medizinischen, insbesondere zahnärztlichen, Systems (1) angeordnet ist, um elektrische Signale und/ oder Daten zwischen dem Zubehör (34) und dem medizinischen, insbesondere zahnärztlichen, Hand- oder Winkelstück (5, 8) zu übertragen, wobei das Zubehör (34) als Brille (34) ausgebildet ist.

2. Medizinisches, insbesondere zahnärztliches, System (1) nach Anspruch 1, **dadurch gekennzeichnet, dass**
die zumindest eine Sendeeinheit (74) und die zumindest eine Empfangseinheit (77) jeweils zumindest eine elektrische Leiterplatte (83, 84) zum Erzeugen und/ oder Empfangen von elektromagnetischen Feldern, welche kapazitiv in den menschlichen Körper (81) ein- bzw. auskoppelbar sind, mit einem isolierten Basiskörper (85) und zumindest einer elektrischen Leiterbahn (86) aufweisen.

3. Medizinisches, insbesondere zahnärztliches, System (1) nach Anspruch 2, **dadurch gekennzeichnet, dass**
der Basiskörper (85) der elektrischen Leiterplatte (83, 84) ein flexibles Trägermaterial, vorzugsweise eine Trägerfolie, aufweist.

4. Medizinisches, insbesondere zahnärztliches, System (1) nach Anspruch 1, 2 oder 3, **dadurch gekennzeichnet, dass**
die Brille (34) eine Schutzbrille aufweist.

5. Medizinisches, insbesondere zahnärztliches, System (1) nach Anspruch 1, 2, 3 oder 4, **dadurch gekennzeichnet, dass**
die zumindest eine Sendeeinheit (74) und/ oder die zumindest eine Empfangseinheit (77) zumindest einen Energiespeicher (78) zur Versorgung der Sendeeinheit (74) und/ oder der Empfangseinheit (77) mit elektrischer Energie und zumindest eine Speichereinheit (79) zum Speichern und/ oder Ausgeben von Daten aufweist/ aufweisen.

6. Medizinisches, insbesondere zahnärztliches, System (1) nach einem der vorherigen Ansprüche, **dadurch gekennzeichnet, dass**
die zumindest eine Sendeeinheit (74) und/ oder die zumindest eine Empfangseinheit (77) jeweils in einem Gehäuse (80) angeordnet ist/ sind, wobei das Gehäuse (80) derart beschaffen ist, dass die Funktionsfähigkeit der in dem Gehäuse (80) angeordneten Sende- oder Empfangseinheit (74, 77) auch nach mehrmaliger Reinigung, Pflege und/ oder Sterilisation aufrechterhaltbar ist.

7. Medizinisches, insbesondere zahnärztliches, System (1) nach einem der vorherigen Ansprüche, **dadurch gekennzeichnet, dass**
die Brille (34) als Filterbrille zur Erkennung einer Anomalie an einem Zahn ausgebildet ist.

8. Medizinisches, insbesondere zahnärztliches, System (1) nach Anspruch 7, **dadurch gekennzeichnet, dass**
die Filterbrille (34) zwei Filter (88, 89) aufweist, die ausgebildet sind, von einem zu untersuchenden Zahn abgestrahlte Strahlung durchzulassen, deren Wellenlänge zum Nachweis der Anomalie an einem Zahn geeignet ist.

9. Medizinisches, insbesondere zahnärztliches, System (1) nach einem der vorherigen Ansprüche, **dadurch gekennzeichnet, dass**
die zumindest eine Empfangseinheit (77) in oder an einem Griff- und/ oder Haltestück (6) des Hand- oder Winkelstücks (5, 8) angeordnet ist.

10. Medizinisches, insbesondere zahnärztliches, System (1) nach einem der vorherigen Ansprüche, **dadurch gekennzeichnet, dass**
die zumindest eine Empfangseinheit (77) in oder an einem Betätigungs- und/ oder Bedienelement des Hand- oder Winkelstücks angeordnet ist.

11. Medizinisches, insbesondere zahnärztliches, System (1) nach einem der vorherigen Ansprüche, **dadurch gekennzeichnet, dass**
das medizinische, insbesondere zahnärztliche, Hand- oder Winkelstück (5, 8) eine Lichtquelle (7) mit zumindest einer ersten Leuchtdiode zur Beleuchtung einer Behandlungsstelle und zumindest einer zweiten Leuchtdiode zur Erkennung einer Anomalie an einer Behandlungsstelle aufweist, wobei die erste und zwei Leuchtdiode von einer Antriebs- und/ oder Steuereinheit (12) wahlweise zur Beleuchtung der Behandlungsstelle oder zur Erkennung einer Anomalie ansteuerbar sind.

12. Medizinisches, insbesondere zahnärztliches, System (1) nach Anspruch 11, **dadurch gekennzeichnet, dass**
die Antriebs- und/ oder Steuereinheit (12) zum Empfang eines elektrischen Signals von der Sendeeinheit (74) der Filterbrille (34) zur automatischen Auswahl der ersten Leuchtdiode oder der zweiten Leuchtdiode ausgebildet ist, wobei die Empfangseinheit (77) des Handstück (5, 8) ausgebildet ist, das von der Sendeeinheit (74) der Filterbrille (34) über den Pfad (82) geleitete elektrische Signal zu empfangen, so dass das elektrische Signal an die Antriebs- und/ oder Steuereinheit (12) weiterleitbar ist, so dass die Antriebs- und/ oder Steuereinheit (12) automatisch die zweite Leuchtdiode zur Erkennung der Anomalie an einem Zahn aktiviert.

13. Medizinisches, insbesondere zahnärztliches, System (1) nach einem der vorherigen Ansprüche, **dadurch gekennzeichnet, dass**
das Zubehör als Identifizierungselement (53, 55) zur Benutzererkennung ausgebildet ist.

14. Medizinisches, insbesondere zahnärztliches, System (1) nach einem der Ansprüche 11 - 13, **dadurch gekennzeichnet, dass**
die zumindest eine zweite Leuchtdiode elektromagnetische Strahlung emittiert, die eine oder mehrere Wellenlängen im Bereich von etwa 380 nm bis 420 nm umfasst.

15. Medizinisches, insbesondere zahnärztliches, System (1) nach einem der Ansprüche 7 - 14, **dadurch gekennzeichnet, dass**
die Sendeeinheit (74) in einem Bügel (39) in der Filterbrille (34) angeordnet ist.

## Claims

1. A medical, in particular dental system (1) comprising: a medical, in particular dental, straight handpiece or contra-angled handpiece (5, 8), at least one transmitting unit (74) and at least one receiving unit (77), wherein the at least two units (74, 77) are configured for capacitive coupling of electrical signals and/ or data into and/or out of a human body (81), so that an electric path (82) for the transmission of electric signals and/ or data between the at least two units (74, 74) can be established which extends through the human body (81) of the user and/or of the person to be treated, **characterized in that**
the at least one receiving unit (77) is disposed in or on the medical, in particular dental, straight handpiece or contra-angled handpiece (5, 8) which is configured to drive a tool which works on a treatment site and/or to emit radiation, fluids, energy, and/or abrasive media and/or to provide measurement data, and that the at least one transmitting unit (74) is disposed in or on at least one accessory (34) of the medical, in particular dental system (1) and which is configured for operation with the straight handpiece or contra-angled handpiece (5, 8) in order to transmit electrical signals and/ or data between the accessory (34) and the medical, in particular dental, straight handpiece or contra-angled handpiece (5, 8), wherein the accessory (34) is configured as glasses (34).

2. The medical, in particular dental system (1) according to claim 1, **characterized in that**
the at least one transmitting unit (74) and the at least one receiving unit (77) each comprises at least one electrical printed circuit board (83, 84) having an insulated basic body (85) and at least one electrical conductor (86) for generating and/or receiving electromagnetic fields which can be coupled into and/or out of the human body (81).

3. The medical, in particular dental, system (1) according to claim 2, **characterized in that**
the base body (85) of the printed circuit board (83, 84) comprises a flexible carrier material, preferably a carrier film.

4. The medical, in particular dental system (1) according to claims 1, 2 or 3, **characterized in that**
the glasses (34) comprise protective goggles.

5. The medical, in particular dental system (1) according to claims 1, 2, 3 or 4, **characterized in that**
the at least one transmitting unit (74) and/or the at least one receiving unit (77) comprise(s) at least one energy storage system (78) to supply the transmitting unit (74) and/or receiving unit (77) with electric energy and at least one memory unit (79) to store and/or put out data.

6. The medical, in particular dental system (1) according to one of the preceding claims, **characterized in that**
the at least one transmitting unit (74) and/or the at least one receiving unit (77) each is/ are disposed in a housing (80), wherein the housing (80) is designed so that the functionality of the transmitting or receiving unit (74, 77), disposed within the housing, can be maintained even after multiple processes of cleaning, care, and/or sterilization.

7. The medical, in particular dental system (1) according to one of the preceding claims, **characterized in that**
the glasses (34) are configured as filter glasses to detect an anomaly on a tooth.

8. The medical, in particular dental system (1) according to claim 7, **characterized in that**
the filter glasses (34) comprise two filters (88, 99), which are configured to allow radiation emitted from a tooth to be examined to pass through, the wavelength of the radiation being suitable for detecting an anomaly on a tooth.

9. The medical, in particular dental system (1) according to one of the preceding claims, **characterized in that**
the at least one receiving unit (77) is disposed in or at a handle part and/or holding part (6) of the straight handpiece or contra-angled handpiece (5, 8).

10. The medical, in particular dental, system (1) according to one of the preceding claims, **characterized in that**
the at least one receiving unit (77) is disposed in or at an actuating and/or operating element of the straight handpiece or contra-angled handpiece.

11. The medical, in particular dental system (1) according to one of the preceding claims, **characterized in that**
the medical, in particular dental, straight handpiece or contra-angled handpiece (5, 8) comprises a light source (7) having at least one first light-emitting diode for the illumination of a treatment site and at least one second light-emitting diode for detection of an anomaly at a treatment site, wherein the first and second light-emitting diode are selectively controllable by a drive unit and/or control unit (12) either to illuminate the treatment site or to detect an anomaly.

12. The medical, in particular dental system (1) according to claim 11, **characterized in that**
the drive unit and/or control unit (12) is configured to receive an electrical signal of the transmitting unit (74) of the filter glasses (34) to automatically select the first light-emitting diode or the second light-emitting diode, wherein the receiving unit (77) of the handpiece (5, 8) is configured to receive the electrical signal transmitted by the transmitting unit (74) of the filter glasses (34) via path (82), so that the electrical signal can be further transmitted to the drive unit and/or control unit (12), so that the drive unit and/or control unit (12) automatically activates the second light-emitting diode for detection of an anomaly at a tooth.

13. The medical, in particular dental system (1) according to one of the preceding claims, **characterized in that**
the accessory is configured as identification element (53, 55) for user identification.

14. The medical, in particular dental system (1) according to one of claims 11 - 13, **characterized in that**
the at least one second light-emitting diode emits electromagnetic radiation which comprises one or more wavelengths ranging from about 380 nm to 420 nm.

15. The medical, in particular dental system (1) according to one of claims 7 - 14, **characterized in that**
the transmitting unit (74) is arranged in a temple (39) in the filter glasses (34).

## Revendications

1. Système médical, en particulier dentaire (1), comprenant: une pièce à main ou pièce coudée médicale, en particulier dentaire (5, 8), au moins une unité d'émission (74) et au moins une unité de réception (77), dans lequel les au moins deux unités (74, 77) sont réalisées pour l'injection de signaux électriques et/ou de données dans un corps humain (81) et/ou pour les en faire sortir de manière capacitive, de sorte qu'il est possible d'établir un chemin électrique (82) pour la transmission de signaux électriques et/ou de données entre les au moins deux unités (74, 77), lequel chemin s'étend à travers le corps humain (81) de l'utilisateur et/ou de la personne à traiter, **caractérisé en ce que**
l'au moins une unité de réception (77) est disposée dans ou sur la pièce à main ou pièce coudée médicale, en particulier dentaire (5, 8) qui est réalisée pour actionner un outil agissant sur un emplacement à traiter et/ou pour émettre un rayonnement, des fluides, de l'énergie et/ou des milieux abrasifs et/ou pour mettre à disposition des données de mesure, et que l'au moins une unité d'émission (74) est disposée dans ou sur au moins un accessoire (34) réalisé pour le fonctionnement de la pièce à main ou pièce coudée (5, 8) du système médical, en particulier dentaire (1) afin de transmettre des signaux électriques et/ou données entre l'accessoire (34) et la pièce à main ou pièce coudée médicale, en particulier dentaire (5, 8), dans lequel l'accessoire (34) est réalisé en tant que lunettes.

2. Système médical, en particulier dentaire (1) selon la revendication 1, **caractérisé en ce que**
l'au moins une unité d'émission (74) et l'au moins une unité de réception (77) présentent respectivement au moins une carte de circuit imprimé électrique (83, 84) pour la génération et/ou réception de champs électromagnétiques que l'on peut injecter dans le corps humain (81) et/ou les en faire sortir de manière capacitive, avec un corps de base isolé (85) et au moins une piste conductrice électrique (86).

3. Système médical, en particulier dentaire (1) selon la revendication 2, **caractérisé en ce que**
le corps de base (85) de la carte de circuit imprimé électrique (83, 84) présente un matériau de support flexible, de préférence un film de support.

4. Système médical, en particulier dentaire (1) selon la revendication 1, 2 ou 3, **caractérisé en ce que**
les lunettes (34) présentent des lunettes de protection.

5. Système médical, en particulier dentaire (1) selon la revendication 1, 2, 3 ou 4, **caractérisé en ce que**
l'au moins une unité d'émission (74) et/ou l'au moins une unité de réception (77) présente/présentent au moins un accumulateur d'énergie (78) pour l'alimentation de l'unité d'émission (74) et/ou de l'unité de réception (77) en énergie électrique et au une unité de mémoire (79) pour stocker et/ou faire sortir des données.

6. Système médical, en particulier dentaire (1) selon l'une des revendications précédentes, **caractérisé en ce que**
l'au moins une unité d'émission (74) et/ou l'au moins une unité de réception (77) est/sont respectivement disposée(s) dans un boîtier (80), dans lequel le boîtier (80) est conçu de telle sorte que la capacité de fonctionnement de l'unité d'émission ou de réception (74, 77) disposée dans le boîtier (80) peut être maintenue également après plusieurs nettoyages, entretiens et/ou stérilisations.

7. Système médical, en particulier dentaire (1) selon l'une des revendications précédentes, **caractérisé en ce que**
les lunettes (34) sont réalisées en tant que lunettes à filtres pour reconnaître une anomalie au niveau d'une dent.

8. Système médical, en particulier dentaire (1) selon la revendication 7, **caractérisé en ce que**
les lunettes à filtres (34) présentent deux filtres (86, 89) réalisés pour laisser passer un rayonnement, émis par une dent à contrôler, dont la longueur d'onde convient pour prouver l'anomalie au niveau d'une dent.

9. Système médical, en particulier dentaire (1) selon l'une des revendications précédentes, **caractérisé en ce que**
l'au moins une unité de réception (77) est disposée dans ou sur une pièce de préhension et/ou de maintien (6) de la pièce à main ou pièce coudée (5, 8).

10. Système médical, en particulier dentaire (1) selon l'une des revendications précédentes, **caractérisé en ce que**
l'au moins une unité de réception (77) est disposée dans ou sur un élément d'actionnement et/ou de commande de la pièce à main ou pièce coudée.

11. Système médical, en particulier dentaire (1) selon l'une des revendications précédentes, **caractérisé en ce que**
la pièce à main ou pièce coudée médicale, en particulier dentaire (5, 8) présente une source lumineuse (7) avec au moins une première diode électroluminescente pour l'éclairage d'un emplacement de traitement et au moins une deuxième diode électroluminescente pour la reconnaissance d'une anomalie au niveau d'un emplacement de traitement, dans lequel les première et deuxième diodes électroluminescentes peuvent être commandées au choix par une unité d'actionnement et/ou de commande (12) pour l'éclairage de l'emplacement de traitement ou la reconnaissance d'une anomalie.

12. Système médical, en particulier dentaire (1) selon la revendication 11, **caractérisé en ce que**
l'unité d'actionnement et/ou de commande (12) est réalisée pour la réception d'un signal électrique de l'unité d'émission (74) des lunettes à filtres (34) pour la sélection automatique de la première diode électroluminescente ou de la deuxième diode électroluminescente, dans lequel l'unité de réception (77) de la pièce à main (5, 8) est réalisée pour réceptionner le signal électrique conduit par l'unité d'émission (74) des lunettes à filtres (34) via le chemin (82) de sorte que le signal électrique puisse être retransmis à l'unité d'actionnement et/ou de commande (12) de sorte que l'unité d'actionnement et/ou de commande (12) active automatiquement la deuxième diode électroluminescente pour la reconnaissance de l'anomalie au niveau d'une dent.

13. Système médical, en particulier dentaire (1) selon l'une des revendications précédentes, **caractérisé en ce que**
l'accessoire est réalisé en tant qu'élément d'identification (53, 55) pour la reconnaissance d'utilisateur.

14. Système médical, en particulier dentaire (1) selon l'une des revendications 11-13, **caractérisé en ce que**
l'au moins une deuxième diode électroluminescente émet un rayonnement électromagnétique qui comprend une ou plusieurs longueurs d'onde dans la plage de près de 380 nm à 420 nm.

15. Système médical, en particulier dentaire (1) selon l'une des revendications 7-14, **caractérisé en ce que**
l'unité d'émission (74) est disposée dans une branche (39) dans les lunettes à filtres (34).
